(19) Europäisches Patentamt
European Patent Office
Office européen des brevets

(11) Veröffentlichungsnummer: **0 003 726**
**B1**

# (12) EUROPÄISCHE PATENTSCHRIFT

(45) Veröffentlichungstag der Patentschrift: **20.01.82**

(51) Int. Cl.³: **C 07 D 307/90, B 41 M 5/12**

(21) Anmeldenummer: **78100862.8**

(22) Anmeldetag: **09.09.78**

## (54) Substituierte Diaminophthalide, Verfahren zu ihrer Herstellung und ihre Verwendung als Farbbildner in druckempfindlichen oder wärmeempfindlichen Aufzeichnungsmaterialien.

(30) Priorität: **10.02.78 CH 1513/78**

(43) Veröffentlichungstag der Anmeldung:
**05.09.79 Patentblatt 79/18**

(45) Bekanntmachung des Hinweises auf die Patenterteilung:
**20.01.82 Patentblatt 82/3**

(84) Benannte Vertragsstaaten:
**BE CH DE FR GB**

(56) Entgegenhaltungen:
**DE - A - 2 537 776**
**US - A - 2 997 481**

(73) Patentinhaber: **CIBA-GEIGY AG**
***Patentabteilung Postfach***
**CH-4002 Basel (CH)**

(72) Erfinder: **Burri, Peter, Dr.**
**Haselmatte 2**
**D-4153 Reinach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Substituierte Diaminophthalide, Verfahren zu ihrer Herstellung und ihre Verwendung als Farbbildner in druckempfindlichen oder wärmeempfindlichen Aufzeichnungsmaterialien

Die vorliegende Erfindung betrifft neue substituierte Diaminophthalide, Verfahren zu ihrer Herstellung und ihre Verwendung als Farbbildner in druckempfindlichen oder wärmeempfindlichen Aufzeichnungsmaterialien.

Die neuen substituierten Diaminophthalide entsprechen der allgemeinen Formel

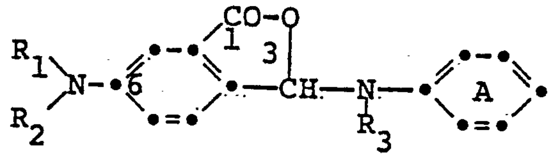

(1)

worin $R_1$, $R_2$ und $R_3$ unabhängig voneinander unsubstituiertes oder durch Halogen, Hydroxyl, Cyano oder Niederalkoxy substituiertes Alkyl mit höchstens 12 Kohlenstoffatomen, Cycloalkyl, Benzyl oder durch Halogen, Niederalkyl oder Niederalkoxy substituiertes Benzyl sind und $R_1$ auch Wasserstoff und $R_3$ auch Phenyl sein können oder $R_1$ und $R_2$ zusammen mit dem sie verbindenden Stickstoffatom einen 5- oder 6-gliedrigen, vorzugsweise gesättigten, heterocyclischen Rest bedeuten und der Ring A unsubstituiert oder durch Halogen, Nitro, Cyano, Trifluormethyl, Niederalkyl, Niederalkoxy, Niederalkoxycarbonyl, Phenyl, Phenoxy, Halogenphenoxy, Di-(Niederalkyl-)amino, Phenylamino, Diphenylamino, N-Niederalkyl-N-phenylamino, Acyl oder Acylamino jeweils mit 1 bis 9 Kohlenstoffatomen, Di-(Niederalkyl-)amino-sulfonyl, Di-(Niederalkyl)-amino-carbonyl, Phenylaminosulfonyl oder Phenylaminocarbonyl substituiert ist oder einen ankondensierten, unsubstituierten oder durch Halogen, Cyano, Nitro, Niederalkyl oder Niederalkoxy substituierten Benzolring aufweist.

Niederalkyl und Niederalkoxy stellen bei der Definition der Reste der Diaminophthalide in der Regel solche Gruppen oder Gruppenbestandteile dar, die 1 bis 5, insbesondere 1 bis 3 Kohlenstoffatome aufweisen, wie z.B. Methyl, Aethyl, n-Propyl, Isopropyl, n-Butyl, sek-Butyl, tert-Butyl oder Amyl bzw. Methoxy, Aethoxy oder Isopropoxy.

Der Acylrest ist besonders Formyl oder Niederalkylcarbonyl wie z.B. Acetyl, Propionyl, oder Benzoyl. Weitere Acylreste sind Niederalkylsulfonyl wie z.B. Methylsulfonyl oder Aethylsulfonyl sowie Phenylsulfonyl. Phenyl, Benzoyl und Phenylsulfonyl können durch Halogen, Methyl, Methoxy oder Aethoxy substituiert sein.

Stellen die Substituenten $R_1$, $R_2$ und $R_3$ Alkylgruppen dar, so können sie geradkettig oder verzweigt sein. Beispiele für solche Alkylreste sind Methyl, Aethyl, n-Propyl, Isopropyl, n-Butyl, sek-Butyl, n-Hexyl, n-Octyl oder n-Docecyl.

Sind die Alkylreste in $R_1$ und $R_2$ substituiert, so handelt es sich vor allem um Cyanoalkyl, Halogenalkyl, Hydroxyalkyl, Alkoxyalkyl jeweils mit 2 bis 4 Kohlenstoffatomen, wie z.B. $\beta$-Cyanoäthyl, $\beta$-Chloräthyl, $\beta$-Hydroxyäthyl, $\beta$-Methoxyäthyl oder $\beta$-Aethoxyäthyl.

Beispiele für Cycloalkyl in der Bedeutung der R-Reste sind Cyclopentyl oder vorzugsweise Cyclohexyl. Bevorzugte Substituenten in der Benzylgruppe der R-Reste sind z.B. Halogen, Methyl oder Methoxy. Beispiele für derartige araliphatische Reste sind p-Methylbenzyl, o- oder p-Chlorbenzyl, oder o- oder p-Methoxybenzyl.

Wenn die Substituenten $R_1$ und $R_2$ zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino.

Die Reste $R_1$ und $R_2$ können voneinander verschieden sein oder sind vorzugsweise identisch. $R_1$ und $R_2$ sind vorzugsweise Benzyl oder Niederalkyl Der Rest $R_3$ ist vorteilhafterweise unsubstituiertes oder durch Halogen, Hydroxyl, Cyano oder Niederalkoxy substituiertes Alkyl mit höchstens 12 Kohlenstoffatomen, Cycloalkyl, Benzyl oder durch Halogen, Niederalkyl oder Niederalkoxy substituiertes Benzyl und insbesondere Niederalkyl oder Benzyl.

Der Ring A ist vorzugsweise definitionsgemäss substituiert. Im Ring A können vorteilhafterweise 1 oder 2 Substituenten vorhanden sein. Bevorzugte Substituenten des Rings A sind Halogen, Nitro, Cyano, Trifluormethyl, Niederalkyl, Niederalkoxy, Phenoxy, Halogenphenoxy, Di-Niederalkylamino, Niederalkylcarbonylamino, Benzoylamino, Niederalkoxy-carbonyl, Di-(Niederalkyl)aminosulfonyl, Di-(Niederalkyl)aminocarbonyl oder ankondensierte, unsubstituierte oder substituierte Benzolringe. Die ankondensierten Benzolringe sind definitionsgemäss substituierte. Besonders bevorzugt enthält der Ring A Halogen, Niederalkyl, Niederalkoxy oder Phenoxy.

Praktisch wichtige Farbbildner der substituierten Diaminophthalide entsprechen der Formel

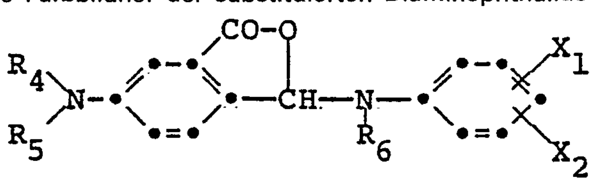

(2)

worin $R_4$, $R_5$ und $R_6$ unabhängig voneinander, Niederalkyl, oder Benzyl oder $R_4$ und $R_5$ zusammen mit dem sie verbindenden Stickstoffatom einen 5- oder 6-gliedrigen, gesättigten heterocyclischen Rest, $X_1$ Halogen, Niederalkyl, Niederalkoxy, Di-(Niederalkyl)amino, Phenoxy, Halogenphenoxy oder Niederalkylcarbonylamino und $X_2$ Wasserstoff, Halogen, Niederalkyl oder Niederalkoxy bedeuten.

Von ganz besonderem Interesse sind substituierte 3,6-Diaminophthalide der Formel

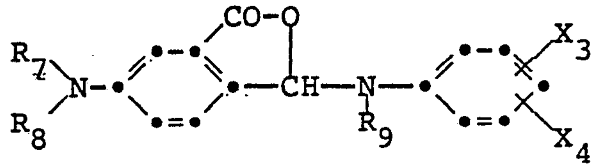

(3)

worin $R_7$, $R_8$ und $R_9$ unabhängig voneinander, Niederalkyl, $X_3$ Halogen, Niederalkyl, Niederalkoxy oder Phenoxy und $X_4$ Wasserstoff, Halogen, Niederalkyl oder Niederalkoxy bedeuten.

Unter diesen Diaminophthaliden der Formel (3) sind diejenigen besonders bevorzugt, bei denen $R_7$, $R_8$ und $R_9$, unabhängig voneinander, Methyl oder Aethyl, $X_3$ Halogen, Niederalkyl oder Niederalkoxy und $X_4$ Wasserstoff, Halogen, Methyl oder Methoxy bedeuten.

Halogen in Verbindung mit den vorstehenden Substituenten in Formeln (1), (2) und (3) sind beispielsweise Fluor, Brom oder vorzugsweise Chlor.

Die erfindungsgemässen substituierten Diaminophthalide werden dadurch hergestellt, dass man eine Aminophthaldehydsäure der Formel

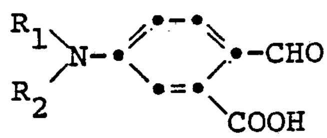

(4)

mit einer Aminoverbindung der Formel

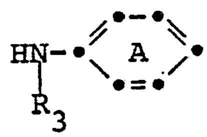

(5)

umsetzt, worin A, $R_1$, $R_2$ und $R_3$ die angegebene Bedeutung haben.

Die Reaktion erfolgt zweckmässig in einem organischen Lösungsmittel und bei Rückflusstemperatur. Basische Kondensationsmittel z.B. Stickstoffbasen wie Pyridin können mit verwendet werden.

Als Lösungsmittel kommen beispielsweise in Betracht: cycloaliphatische oder aromatische Kohlenwasserstoffe wie z.B. Cyclohexan, Benzol, Toluol, Xylol oder Tetrahydronaphthalin; Chlorkohlenwasserstoffe wie z.B. Chloroform, Tetrachlorkohlenstoff, Aethylenchlorid oder Chlorbenzole; niedere aliphatische Alkohole, wie z.B. Methanol, Aethanol oder Isopropanol; Aether wie Dioxan, Diäthyläther, Glykoldimethyläther oder Tetrahydrofuran.

Die Konzentration der Reaktionsteilnehmer ist nicht kritisch; man verwendet jedoch mit Vorteil je ein Moläquivalent der Reaktionskomponenten. Sofern erwünscht, kann das Reaktionsprodukt der Formel (1) noch gereinigt werden, indem man es in organischen Lösungsmitteln umkristallisiert.

Die Amihnophthaldehydsäuren der Formel (4) sind in der DT—OS 2 643 569 beschrieben. Sie können durch Verseifung der entsprechenden Säureester oder durch Umsetzung eines Aminobenzoesäureanhydrids mit Formamiden in Gegenwart eines Säurehalogenids hergestellt werden.

Die substituierten Phthalidverbindungen der Formeln (1) bis (3) sind normalerweise farblos oder schwach gefärbt. Wenn diese Farbbildner mit einem sauren Entwickler, d.h. einem Elektronenakzeptor in Kontakt gebracht werden, so ergeben sie intensive gelbe, orange oder rote Farbtöne, die ausgezeichnet lichtecht sind. Sie sind deshalb auch sehr wertvoll im Gemisch mit einem oder mehreren anderen Farbbildnern, z.B. 3,3-(Bis-aminophenyl)-phthaliden, 3,3-(Bisindolyl-)phthaliden, 2,6-Diaminofluoranen oder Spiropyranen um blaue, marineblaue, graue oder schwarze Färbungen zu ergeben.

Die Phthalidverbindungen der Formel (1) bis (3) zeigen sowohl auf Ton als auch auf phenolischen Unterlagen eine verbesserte Farbintensität und Lichtechtheit. Sie eignen sich vor allem als sich schnell entwickelnde Farbbildner für die Verwendung in einem druckempfindlichen Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner der Formeln (1) bis (3) gelöst in einem organischen Lösungsmittel und einen festen Elektronenakzeptor als Entwickler enthalten. Der Farbbildner liefert an den Punkten, an denen er mit dem Elektronenakzeptor in Kontakt kommt, eine gefärbte Markierung.

Typische Beispiele für solche Entwickler sind Attapulgus-Ton, säureaktiviertes Bentonit (Silton-Ton), Montmorillonit, Siliciumdioxyd, Bentonit, Halloysit, Aluminiumoxyd, Aluminiumsulfat,

Aluminiumphosphat, Zinkchlorid, Kaolin, Tone oder sauer reagierende, organische Verbindungen, wie z.B. gegebenenfalls ringsubstituierte Phenole, Salicylsäure, Salicylsäureester und deren Metallsalze, ferner ein sauer reagierendes, polymeres Material, wie z.B. ein phenolisches Polymerisat, ein Alkylphenolacetylenharz, ein Maleinsäure/Kolophonium-Harz oder ein teilweise oder vollständig hydrolysiertes Polymerisat von Maleinsäureanhydrid mit Styrol, Aethylen, Vinylmethyläther oder Carboxy-Polymethylen. Bevorzugte Entwickler sind Attapulgus-Ton, Silton-Ton, Zinksalicylate oder Phenolformaldehydharze. Diese Elektronenakzeptoren werden vorzugsweise in Form einer Schicht auf die Vorderseite des Empfangsblattes aufgebracht.

Um zu verhindern, dass in dem druckempfindlichen Aufzeichnungsmaterial die Farbbildner frühzeitig aktiv werden, trennt man sie in der Regel von dem Elektronenakzeptor. Dies kann zweckmässig erzielt werden, indem man die Farbbildner in schaum-, schwamm- oder bienenwabenartige Strukturen einarbeitet. Vorzugsweise sind die Farbbildner in Mikrokapseln eingeschlossen, die sich in der Regel zerbrechen lassen.

Wenn die Kapseln durch Druck, beispielsweise mittels eines Bleistiftes zerbrochen werden und dadurch die Farbbildnerlösung auf ein benachbartes Blatt übertragen wird, das mit einem Elektronenakzeptor beschichtet ist, wird eine farbige Stelle erzeugt. Diese Farbe ergibt sich aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. polyhalogenisiertes Paraffin oder Diphenyl, wie Trichlordiphenyl oder eine Mischung davon mit flüssigem Paraffin, ferner Tricresylphosphat, Di-n-butylphthalat, Dioctylphthalat, Trichlorbenzol, Nitrobenzol, Trichloräthylphosphat, Kohlenwasserstofföle wie Paraffin, alkylierte Derivate von Diphenyl, Naphthalin oder Triphenyl, Terphenyle, partielle hydriertes Terphenyl oder weitere chlorierte oder hydrierte, kondensierte, aromatische Kohlenwasserstoffe.

Die Kapselwände können durch Koazervationskräfte gleichmässig um die Tröpfchen der Farbbildnerlösung herum gebildet werden, wobei das Einkapselungsmaterial z.B. aus Gelatine und Gummiarabicum bestehen kann, wie dies z.B. in der US-Patentschrifft 2 800 457 beschrieben ist. Die Kapseln können vorzugsweise auch aus einem Aminoplast oder modifizierten Aminoplasten durch Polykondensation gebildet werden, wie dies in den britischen Patentschriften 989 264, 1 156 725, 1 301 052 und 1 355 124 beschrieben wird.

Die Farbbildner der Formel (1) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind. Die Komponenten können aber auch in der Papierpulpe verwendet werden.

Eine andere Anordnung besteht darin, dass die Farbbildner enthaltenden Mikrokapseln und der Entwickler in oder auf dem gleichen Blatt in Form einer oder mehrerer Einzelschichten oder in der Papierpulpe vorliegen.

Solche druckempfindliche Kopiermaterialien sind beispielsweise in den US-Patentschriften 2 730 457, 2 932 582, 3 418 250, 3 427 180 und 3 516 846 beschrieben. Weitere Systeme sind in den britischen Patentschriften 1 042 596, 1 042 597, 1 042 598, 1 042 599 und 1 053 935 beschrieben. Mikrokapseln, welche die Farbbildner der Formel (1) enthalten, eignen sich für jedes dieser Systeme sowie für andere druckempfindliche Systeme.

Die Kapseln werden vorzugsweise mittels eines geeigneten Klebstoffes auf dem Träger befestigt. Da Papier das bevorzugte Trägermaterial ist, handelt es sich bei diesen Klebstoffen hauptsächlich um Papierbeschichtungsmittel, wie Gummiarabicum, Polyvinylalkohol, Hydroxymethylcellulose, Casein, Methylcellulose oder Dextrin.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Phthalidverbindungen der Formeln (1) bis (3) können auch als Farbbildner in einem thermoreaktiven Aufzeichnungsmaterial verwendet werden. Dieses enthält in der Regel mindestens einen Träger, einen Farbbildner, einen festen Elektronenakzeptor und gegebenenfalls auch ein Bindemittel. Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Messinstrumenten verwendet. Die Bilderzeugung (Markierungserzeugung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie z.B. 4-tert-Butylphenol, 4-Phenylphenol, 4-Hydroxydiphenyläther, $\alpha$-Naphthol, $\beta$-Naphthol, 4-Hydroxybenzoesäuremethylester, 4-Hydroxyacetophenon, 2,2'-Dihydroxydiphenyl, 4,4'-Isopropylidendiphenol, 4,4'-Isopropylidenbis-(2-methylphenol), 4,4'-Bis-(hydroxyphenyl)valeriansäure, Hydrochinon, Pyrogallol, Phloroglucin, p-, m-, o-Hydroxybenzoesäure, Gallussäure, 1-Hydroxy-2-naphthosäure sowie Borsäure und aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare filmbildende Bindemittel verwendet. Diese Bindemittel sind normalerweise wasserlöslich, während die Phthalidverbindung und der Entwickler in Wasser unlöslich sind. Das Bindemittel sollte in der Lage sein, den Farbbildner und den Entwickler bei Raumtemperatur zu dispergieren und zu fixieren. Bei Einwirkung von Wärme erweicht oder schmilzt das Bindemittel, so dass der Farbbildner mit dem Entwickler in Kontakt kommt und eine Farbe bilden kann. Wasserlösliche oder mindestens in Wasser quellbare Bindemittel sind z.B. hydrophile Polymerisate, wie Polyvinylalkohol, Polyacrylsäure, Hydroxyäthylcellulose, Methylcellulose, Carboxymethylcellulose, Polyacrylamid, Polyvinylpyrrolidon, Gelatine und Stärke.

Wenn der Farbbildner und der Entwickler in zwei getrennten Schichten vorliegen, können in Wasser unlösliche Bindemittel, d.h. in nicht-polaren oder nur schwach polaren Lösungsmitteln lösliche Bindemittel, z.B. Naturkautschuk, synthetischer Kautschuk, chlorierter Kautschuk, Alkydharze, Polystyrol, Styrol/Butadien-Mischpolymerisate, Polymethylacrylate, Aethylcellulose, Nitrocellulose und Polyvinylcarbazol, verwendet werden. Bevorzugt wird eine Anordnung, bei der der Farbbildner und der Entwickler in einer Schicht in einem wasserlöslichen Bindemittel enthalten sind.

Die thermoreaktiven Schichten können weiter Zusätze enthalten. Zur Verbesserung des Weissgrades, zur Erleichterung des Bedruckens der Papiere und zur Verhinderung des Festklebens der erhitzten Feder können diese Schichten, z.B. talk, $TiO_2$, ZnO oder $CaCO_3$ oder auch organische Pigmente, wie z.B. Harnstoff-Formaldehydpolymerisate enthalten. Um zu bewirken, dass nur innerhalb eines begrenzten Temperaturbereiches die Farbe gebildet wird, können Substanzen, wie Harnstoff, Thioharnstoff, Acetanilid, Phthalsäureanhydrid oder andere entsprechende, schmelzbare Produkte, welche das gleichzeitige Schmelzen des Farbbildners und des Entwicklers induzieren, zugesetzt werden.

In den folgenden Beispielen beziehen sich die angegebenen Prozentsätze, wenn nichts anderes angegeben ist, auf das Gewicht.

Beispiel 1

19,3 g 2-Carboxy-4-dimethylamino-benzaldehyd (2-Formyl-5-dimethylaminobenzoesäure) und 16,6 g N-Methyl-p-äthoxy-anilin werden in 200 ml Toluol am Rückfluss erhitzt. Nach 5½ Stundden hat sich alles in der Reaktion gebildete Wasser über einem Wasserabscheider abgetrennt. Man kocht die Lösung mit etwas Tierkohle und filtriert. Nach Zugabe von 100 ml Petroläther kristallisiert das Produkt aus. Nach dem Abfiltrierten und Trocknen in Vakuum bei 60°C erhält man 20,4 g einer Verbindung der Formel

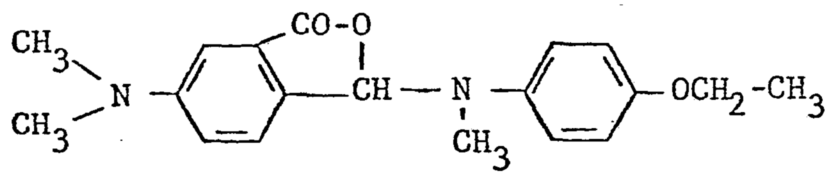

(11)

Die Verbindung schmilzt bei 135—136°C.

Auf Silton-Ton entwickelt dieser Farbbildner eine starke gelbe Färbung mit ausgezeichneter Lichtechtheit.

Beispiel 2

11,6 g 2-Carboxy-4-dimethylamino-benzaldehyd und 8,4 g N-methyl-p-chloranilin werden in 250 ml Toluol zum Rückfluss erhitzt. Ueber einem Wasserabscheider wird das bei der Reaktion gebildete Wasser abgetrennt. Nach 16 Stunden ist die Reaktion beendet. Nun gibt man zur Toluollösung 200 ml Petroläther zu, worauf das Produkt kristallin ausfällt. Nach dem Abfiltrieren und Trocknen in Vakuum bei 60°C erhält man 16,1 g des farblosen Produkts der Formel

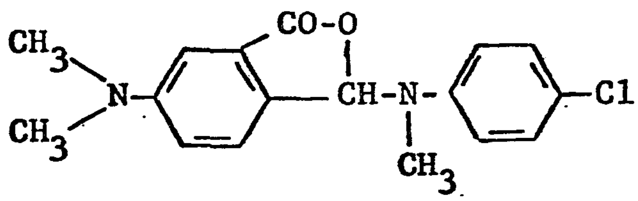

(12)

Die Verbindung hat einen Schmelzpunkt von 152—154°C.

Auf Silton-Ton entwickelt dieser Farbbildner sofort eine starke gelbe Färbung mit ausgezeichneter Lichtechtheit.

Beispiel 3

11,6 g 2-Carboxy-4-dimethylamino-benzaldehyd und 7,3 g N-methyl-p-toluidin werden in 250 ml Toluol am Rückfluss erhitzt. Nach 16 Stunden hat sich alles in der Reaktion gebildete Wasser über einem Wasserabscheider abgetrennt. Die Toluollösung wird mit 200 ml Petroläther verdünnt, worauf sich das Produkt kristallin abscheidet. Nach dem Abfiltrieren und Trocknen in Vakuum bei 60°C erhält man 7,0 g einer farblosen Verbindung der Formel

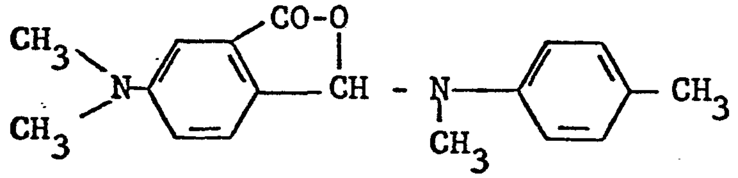

(13)

Die Verbindung schmiltz bei 135—137°C.

Auf Silton-Ton entwickelt dieser Farbbildner sofort eine starke gelbe Färbung mit ausgezeichneter Lichtechtheit.

Ersetzt man in diesem Beispiel das 2-Carboxy-4-dimethylaminobenzaldehyd durch eine äquimolare Menge von 2-Carboxy-4-diäthylaminobenzaldehyd, so erhält man die entsprechende 3-(N-Methyl-p-toluidino)-6-diäthylamino-phthalidverbindung, die auf Silton-Ton ebenfalls eine gelbe Färbung von guter Lichtechtheit ergibt.

Beispiel 4

11,6 g 2-Carboxy-4-dimethylamino-benzaldehyd und 7,3 g N-Aethyl-m-toluidin werden in 250 ml Toluol zum Rückfluss erhitzt. Nach 16 Stunden hat sich alles in der Reaktion gebildete Wasser über einem Wasserabscheider abgetrennt. Die Toluollösung wird mit 200 ml Petroläther verdünnt, worauf sich das Produkt kristallin abscheidet.

Nach dem Filtrierten und Trocknen in Vakuum bei 60°C erhält man 16,1 g einer farblosen Verbindung der Formel

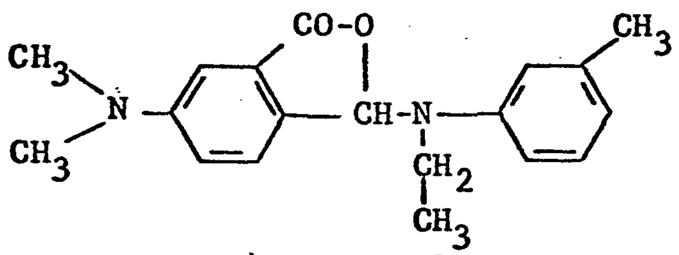

(14)

Die Verbindung hat einen Schmelzpunkt von 138—139°C.

Auf Silton-Ton entwickelt dieser Farbbildner sofort eine starke gelbe Färbung mit ausgezeichneter Lichtechtheit.

Auf gleiche Weise wie in Beispiel 1 beschrieben, erhält man die in der folgenden Tabelle aufgeführten Farbbildner der Formel

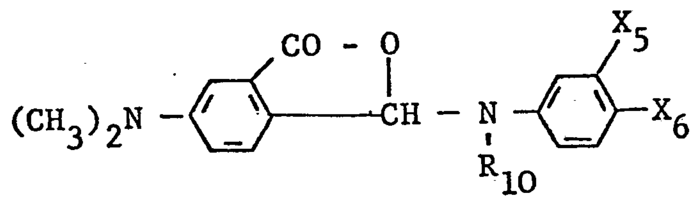

(15)

TABELLE

| Beispiel Nr | $R_{10}$ | $X_5$ | $X_6$ | smp °C | Farbe auf Silton Ton |
|---|---|---|---|---|---|
| 5 | $CH_3$ | H | –O– 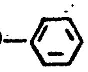 | 125–126 | grünstichig gelb |
| 6 | $CH_3$ | H | $-N(CH_3)_2$ | 142–144 | orange-rot |
| 7 | $CH_3$ | $CF_3$ | H | 137–139 | grünstichig gelb |
| 8 | $CH_3$ | H | $NO_2$ | 244–246 | gelb |
| 9 | $CH_3$ | Cl | Cl | 180–183 | gelb |
| 10 | $CH_3$ | H | Br | 152–154 | gelb |
| 11 | $CH_3$ | $-OCH_3$ | $-OCH_3$ | 139–142 | gelb |
| 12 | $-CH_2-$ 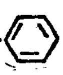 | H | Cl | 144–149 | gelb |
| 13 | $-CH_2CH_3$ | H | Cl | 140–142 | gelb |

Beispiel 14

6,4 g 2-Carboxy-4-dimethylamino-benzaldehyd und 3,9 g N-Methylanilin werden in 150 ml Benzol am Rückfluss erhitzt. Ueber einem Wasserabscheider wird dass in der Reaktion gebildete Wasser abgeschieden. Nach 8 Stunden werden 50 ml Benzol abdestilliert und die Reaktionslösung abgekühlt. Dabei kristallisiert das farblose Produkt aus. Nach dem Abfiltrieren und Trocknen in Vakuum bei 60°C erhält man 7,1 g der Verbindung der Formel

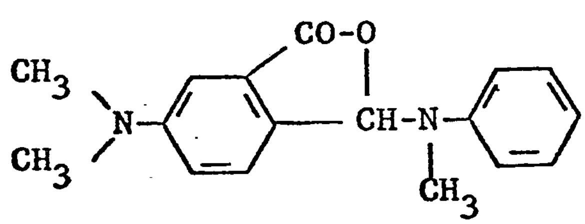

(16)

Die Verbindung schmilzt bei 152°C.

Auf Silton-Ton entwickelt dieser Farbbildner sofort eine grünstichig-gelbe Färbung von ausgezeichneter Lichtechtheit.

Beispiel 15

Herstellung eines druckempfindlichen Kopierpapiers

Eine Lösung von 3 g der Phthalidverbindung der Formel (11) in 97 g partiell hydriertem Terphenyl wird in einer Lösung von 12 g Schweinhautgelatine in 88 g Wasser von 50°C emulgiert. Sodann wird eine Lösung von 12 g Gummiarabicum in 88 g Wasser von 50°C zugegeben und hierauf 200 ml Wasser von 50°C zugefügt. Die erhaltene Emulsion wird in 600 g Eiswasser eingegossen und gekühlt, wobei die Koazervation bewirkt wird. Mit der dabei erhaltenen Suspension der Mikrokapseln wird ein Blatt Papier beschichtet und getrocknet. Ein zweites Blatt Papier wird mit Silton-Ton beschichtet. Das

erste Blatt und das mit Silton-Ton beschichtete Papier werden mit den Beschichtungen benachbart aufeinander gelegt.

Durch Schreiben mit der Hand oder mit der Schreibmaschine auf dem ersten Blatt wird Druck ausgeübt und es entwickelt sich auf dem mit Ton beschichteten Blatt eine intensive gelbe Kopie, die ausgezeichnet lichtecht ist.

Intensive, lichtechte gelbe Kopien können auch erhalten werden, wenn anstelle der Phthalidverbindung der Formel (11) die Farbbildner der Formeln (12) bis (16) eingesetzt werden.

Beispiel 16

Herstellung eines thermoreaktiven Papieres

6 g einer wässerigen Dispersion, die 1,57% der Phthalidverbindung der Formel (11) und 6,7% Polyvinylalkohol enthält, werden mit 134 g einer wässerigen Dispersion gemischt, die 14% 4,4-Isopropylidendiphenol, 8% Attapulgus-Ton und 6% Polyvinylalkohol enthält. Dieses Gemisch wird auf ein Papier aufgetragen und getrocknet. Durch Berührung des Papiers mit einem erhitzten Kugelschreiber wird eine gelbe Farbe erhalten, die eine ausgezeichnete Lichtechtheit hat.

Intensive gelbe Farben können auch bei Verwendung jedes der anderen Farbbildner der Beispiele 2 bis 14 erhalten werden.

**Patentansprüche**

1. Substituierte 3,6-Diaminophthalide, dadurch gekennzeichnet, dass sie der allgemeinen Formel

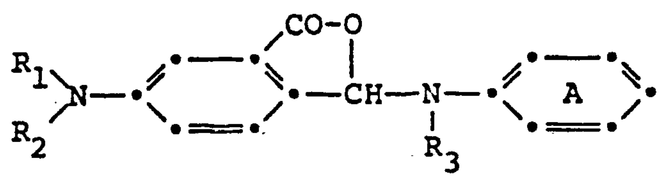

(1)

entsprechen, worin $R_1$, $R_2$ und $R_3$ unabhängig voneinander unsubstituiertes oder durch Halogen, Hydroxyl, Cyano oder Niederalkoxy substituiertes Alkyl mit höchstens 12 Kohlenstoffatomen, Cycloalkyl, Benzyl oder durch Halogen, Niederalkyl oder Niederalkoxy substituiertes Benzyl sind und $R_1$ auch Wasserstoff und $R_3$ auch Phenyl sein können oder $R_1$ und $R_2$ zusammen mit dem sie verbindenden Stickstoffatom einen 5- oder 6-gliedrigen heterocyclischen Rest bedeuten und der Ring A unsubstituiert oder durch Halogen, Nitro, Cyano, Trifluormethyl, Neideralkyl, Niederalkoxy, Niederalkoxycarbonyl, Phenyl, Phenoxy, Halogenphenoxy, Di-(Niederalkyl-)amino, Phenylamino, Diphenylamino, N-Niederalkyl-N-phenylamino, Acyl oder Acylamino jeweils mit 1 bis 9 Kohlenstoffatomen, Di-(Niederalkyl-)amino-sulfonyl, Di-(Niederalkyl)-amino-carbonyl, Phenylaminosulfonyl oder Phenylaminocarbonyl substituiert ist oder einen ankondensierten, unsubstituierten oder durch Halogen, Cyano, Nitro, Niederalkyl oder Niederalkoxy substituierten Benzolring aufweist, wobei niedere Reste solche mit 1 bis 5 C-Atomen sind.

2. Phthalide gemäss Anspruch 1, dadurch gekennzeichnet, dass $R_3$ unsubstituiertes oder durch Halogen, Hydroxyl, Cyano oder Niederalkoxy substituiertes Alkyl mit höchstens 12 Kohlenstoffatomen, Cycloalkyl, Benzyl oder durch Halogen, Niederalkyl oder Niederalkoxy substituiertes Benzyl bedeutet.

3. Phthalide gemäss einem der Ansprüche 1 und 2, dadurch gekennzeichnet, dass der Ring A durch Halogen, Nitro, Cyano, Trifluormethyl, Niederalkyl, Niederalkoxy, Phenoxy, Halogenphenoxy, Di-(Niederalkyl)amino, Niederalkanoylamino, Benzoylamino, Niederalkoxy-carbonyl, Di-(Niederalkyl)aminosulfonyl oder Di-(Niederalkyl)aminocarbonyl substituiert ist oder einen ankondensierten, unsubstituierten oder durch Halogen, Cyano, Nitro, Niederalkyl oder Niederalkoxy substituierten Benzolring aufweist.

4. Phthalide gemäss Anspruch 3, dadurch gekennzeichnet, dass sie der Formel

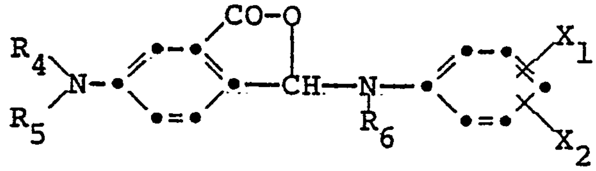

(2)

entsprechen, worin $R_4$, $R_5$ und $R_6$ unabhängig voneinander, Niederalkyl oder Benzyl oder $R_4$ und $R_5$ zusammen mit dem sie verbindenden Stickstoffatom einen 5- oder 6-gliedrigen, gesättigten heterocyclischen Rest, $X_1$ Halogen, Niederalkyl, Niederalkoxy, Di-(Niederalkyl)amino, Phenoxy, Halogenphenoxy oder Niederalkylcarbonylamino und $X_2$ Wasserstoff, Halogen, Niederalkyl oder Niederalkoxy bedeuten.

5. Phthalide gemäss Anspruch 4, dadurch gekennzeichnet, dass sie der Formel

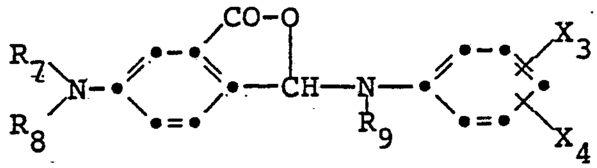

(3)

entsprechen

worin $R_7$, $R_8$ und $R_9$ unabhängig voneinander, Niederalkyl, $X_3$ Halogen, Niederalkyl, Niederalkoxy, oder Phenoxy und $X_4$ Wasserstoff, Halogen, Niederalkyl oder Niederalkoxy bedeuten.

6. Phthalide gemäss Anspruch 5, dadurch gekennzeichnet, dass $R_7$, $R_8$ und $R_9$ unabhängig voneinander Methyl oder Aethyl, $X_3$ Halogen, Niederalkyl oder Niederalkoxy und $X_4$ Wasserstoff, Halogen, Methyl oder Methoxy bedeuten.

7. Verfahren zur Herstellung von substituierten Diaminophthaliden der in Anspruch 1 angegebenen Formel, dadurch gekennzeichnet, dass man eine Aminophthaldehydsäureverbindung der Formel

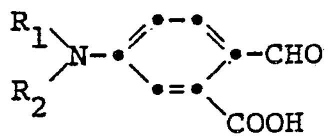

(4)

mit einer Aminoverbindung der Formel

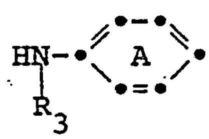

(5)

umsetzt, worin

A, $R_1$, $R_2$ und $R_3$ die im Anspruch 1 angegebene Bedeutung haben.

8. Verwendung einer Phthalidverbindung der in einem der Ansprüche 1 bis 6 angegebenen Formel als Farbbildner in einem druckempfindlichen oder wärmeempfindlichen Aufzeichnungsmaterial.

9. Druck- oder wärmeempfindliches Aufzeichnungsmaterial, dadurch gekennzeichnet, dass es in seinem Farbreaktantensystem als Farbbildner mindestens eine Phthalidverbindung der in einem der Ansprüche 1 bis 6 angegebenen Formel enthält.

10. Druckempfindliches Aufzeichnungsmaterial nach Anspruch 9, dadurch gekennzeichnet, dass es die Phthalidverbindung gelöst in einem organischen Lösungsmittel und mindestens einen festen Elektronenakzeptor enthält.

**Claims**

1. A substituted 3,6-diaminophthalide of the formula

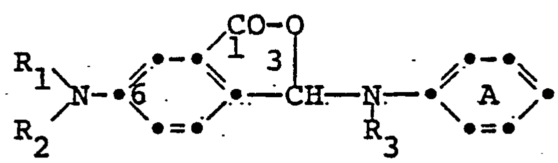

(1)

wherein

each of $R_1$, $R_2$ and $R_3$ independently of the other is alkyl of not more than 12 carbon atoms which is unsubstituted or substituted by halogen, hydroxyl, cyano or lower alkoxy, or is cycloalkyl, benzyl, or benzyl which is substituted by halogen, lower alkyl or lower alkoxy, and $R_1$ can also be hydrogen and $R_3$ can also be phenyl, or $R_1$ and $R_2$, together with the nitrogen atom to which they are attached, are a 5- or 6-membered heterocyclic radical and the ring A is unsubstituted or substituted by halogen, nitro, cyano, trifluoromethyl, lower alkyl, lower alkoxy, lower alkoxycarbonyl, phenyl, phenoxy, halophenoxy, di-(lower alkyl)amino, phenylamino, diphenylamino, N-lower alkyl-N-phenylamino, acyl or acylamino, each containing 1 to 9 carbon atoms, di-(lower alkyl)aminosulfonyl, di-(lower alkyl)aminocarbonyl, phenylaminosulfonyl or phenylaminocarbonyl, or contains a fused benzene ring which is unsubstituted or substituted by halogen, cyano, nitro, lower alkyl or lower alkoxy, the radicals qualified by the term "lower" denoting those containing 1 to 5 carbon atoms.

2. A phthalide according to claim 1, wherein $R_3$ is alkyl of not more than 12 carbon atoms which is unsubstituted or substituted by halogen, hydroxyl, cyano or lower alkoxy, or is cycloalkyl, benzyl, or benzyl which is substituted by halogen, lower alkyl or lower alkoxy.

3. A phthalide according to one of claims 1 and 2, wherein the ring A is substituted by halogen, nitro, cyano, trifluoromethyl, lower alkyl, lower alkoxy, phenoxy, halophenoxy, di-(lower alkyl)amino, lower alkanoylamino, benzoylamino, lower alkoxycarbonyl, di-(lower alkyl)aminosulfonyl or di-(lower alkyl)aminocarbonyl, or contains a fused benzene ring which is unsubstituted or substituted by halogen, cyano, nitro, lower alkyl or lower alkoxy.

4. A phthalide according to claim 3 of the formula

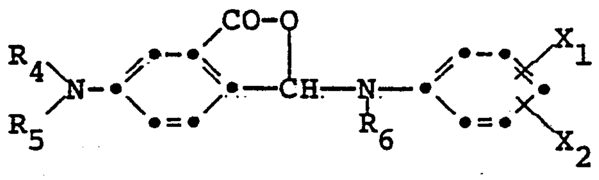

(2)

wherein

each of $R_4$, $R_5$ and $R_6$ independently is lower alkyl or benzyl, or $R_4$ and $R_5$, together with the nitrogen atom to which they are attached, are a saturated 5- or 6-membered heterocyclic radical, $X_1$ is halogen, lower alkyl, lower alkoxy, di-(lower alkyl)amino, phenoxy, halophenoxy or lower alkylcarbonylamino, and $X_2$ is hydrogen, halogen, lower alkyl or lower alkoxy.

5. A phthalide according to claim 4 of the formula

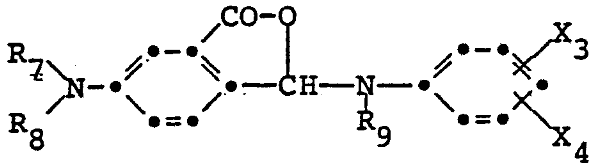

(3)

wherein

each of $R_7$, $R_8$ and $R_9$ independently is lower alkyl, $X_3$ is halogen, lower alkyl, lower alkoxy or phenoxy, and $X_4$ is hydrogen, halogen, lower alkyl or lower alkoxy.

6. A phthalide according to claim 5, wherein each of $R_7$, $R_8$ and $R_9$ independently is methyl or ethyl, $X_3$ is halogen, lower alkyl or lower alkoxy, and $X_4$ is hydrogen, halogen, methyl or methoxy.

7. A process for the production of a substituted diaminophthalide of the formula indicated in claim 1, which process comprises reacting an aminophthaldehyde acid compound of the formula

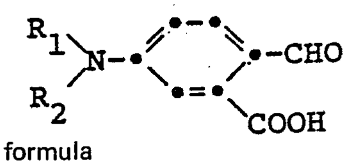

(4)

with an amino compound of the formula

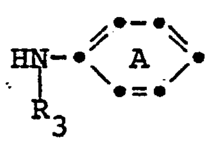

(5)

wherein

A, $R_1$, $R_2$ and $R_3$ are as defined in claim 1.

8. Use of a phthalide compound of the formula as indicated in any one of claims 1 to 6 as colour former in a pressure-sensitive or heat-sensitive recording material.

9. A pressure-sensitive or heat-sensitive recording material which contains as colour former in its colour reactant system at least one phthalide compound of the formula as indicated in any one of claims 1 to 6.

10. A pressure-sensitive recording material according to claim 9, which contains the colour former dissolved in an organic solvent, and at least one solid electron acceptor.

**Revendications**

1. 3,6-diaminophtalides substitués caractérisés en ce qu'ils correspondent à la formule générale:

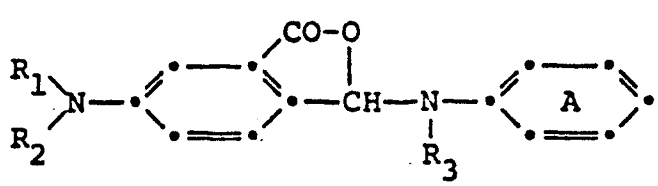

(1)

où $R_1$, $R_2$ et $R_3$ représentent indépendamment les uns des autres un reste alcoyle comportant au maximum 12 atomes de carbone, non substitué ou bien substitué par un halogène, un hydroxyle, un groupe cyano ou alcoxy inférieur, représentent un reste cycloalcoyle, benzyle ou benzyle substitué par un halogène, un alcoyle inférieur ou un alcoxy inférieur et $R_1$ peut représenter aussi de l'hydrogène et $R_3$ peut représenter aussi un radical phényle ou bien $R_1$ et $R_2$ représentent ensemble avec l'atome d'azote qui les relie un reste hétérocyclique à 5 ou 6 chaînons et la noyau A est non substitué ou bien est substitué par un halogène, un groupe nitro, cyano, trifluorométhyle, alcoyle inférieur, alcoxy inférieur, alcoxy-carbonyle inférieur, phényle, phénoxy, halogénophénoxy, di-(alcoyl inférieur)-amino, phénylamino, diphénylamino, N-alcoyl inférieur, N-phénylamino acyle ou acylamino comportant dans chaque cas 1 à 9 atomes de carbone, di-(alcoyl inférieur)-amino-sulfonyle, di(alcoyl inférieur)-amino-carbonyle, phénylaminosulfonyle ou bien phénylaminocarbonyle, ou bien comporte un noyau benzénique condensé, non substitué ou bien substitué par un halogène, un groupe cyano, nitro, alcoyle inférieur ou alcoxy inférieur, les restes inférieurs étant des restes comportant 1 à 5 atomes de carbone.

2. Phtalides selon la revendication 1, caractérisés en ce que $R_3$ représente un reste alcoyle comportant au maximum 12 atomes de carbone, non substitué ou bien substitué par un halogène, un groupe hydroxyle, cyano ou alcoxy inférieur, représente un reste cycloalcoyle, benzyle ou bien benzyle substitué par un halogène, un alcoyle inférieur ou un alcoxy inférieur.

3. Phtalides selon les revendications 1 et 2, caractérisés en ce que le noyau A est substitué par un halogène, un groupe nitro, cyano, trifluorométhyle, alcoyle inférieur, alcoxy inférieur, phénoxy, halogénophénoxy, di-(alcoyl inférieur)amino, alcanoylamino inférieur, benzoylamino, alcoxycarbonyle inférieur, di(alcoyl inférieur) aminosulfonyle ou di-(alcoyl inférieur)aminocarbonyle ou bien comporte un résidu benzénique condensé, non substitué ou bien substitué par un halogène, un groupe cyano, nitro, alcoyle inférieur ou alcoxy inférieur.

4. Phtalides selon la revendication 3, caractérisés en ce qu'ils correspondent à la formule:

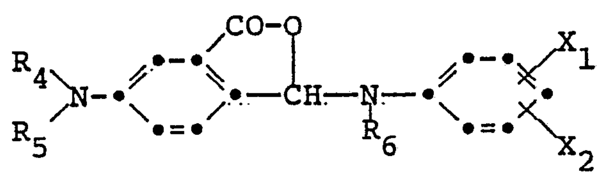

(2)

où $R_4$, $R_5$ et $R_6$ représentent indépendamment les uns des autres un reste alcoyle inférieur ou benzyle ou bien $R_4$ et $R_5$ représentent ensemble avec l'atome d'azote qui les relie un reste hétérocyclique saturé, à 5 ou 6 chaînons, $X_1$ représente un halogène, un reste alcoyle inférieur, alcoxy inférieur, di-(alcoyl inférieur)amino, phénoxy, halogénophénoxy ou bien alcoylcarbonylamino inférieur et $X_2$ représente de l'hydrogène, un halogène, un reste alcoyle inférieur ou alcoxy inférieur.

5. Phtalides selon la revendication 4, caractérisés en ce qu'ils correspondent à la formule:

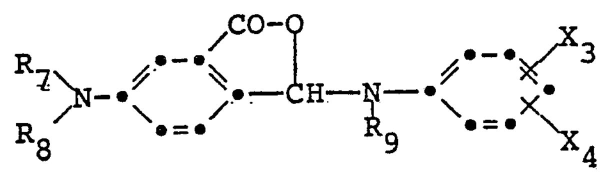

(3)

où $R_7$, $R_8$ et $R_9$ représentent indépendamment les uns des autres un reste alcoyle inférieur, $X_3$ représente un halogène, un reste alcoyl inférieur, alcoxy inférieur ou phénoxy et $X_4$ représente de l'hydrogène, un halogène, un reste alcoyl inférieur ou alcoxy inférieur.

6. Phtalides selon la revendication 5, caractérisés en ce que $R_7$, $R_8$ et $R_9$ représentent indépendamment les uns des autres un reste méthyle ou éthyle, $X_3$ représente un halogène, un reste alcoyle inférieur ou un alcoxy inférieur et $X_4$ représente de l'hydrogène, un halogène, un reste méthyle ou méthoxy.

7. Procédé pour préparer des diaminophtalides substitués de formule indiquée dans la revendication 1, caractérisé en ce qu'on fait réagir un acide aminophtaldéhydique de formule:

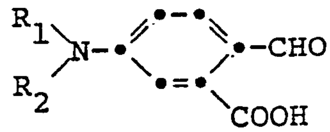

(4)

avec un composé amino de formule:

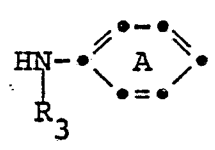

(5)

où A, $R_1$, $R_2$ et $R_3$ ont la signification indiquée dans la revendication 1.

8. Utilisation d'un phtalide de formule indiquée dans l'une quelconque des revendications 1 à 6 en tant que chromogène dans une matière servant à l'enregistrement, sensible à l'effet de la pression ou de la chaleur.

9. Matière servant à l'enregistrement sensible à l'effet de la pression ou de la chaleur, caractérisée en ce qu'elle renferme dans son système de réactifs colorés, en tant que chromogène, au moins un phtalide de formule indiquée dans l'une quelconque des revendications 1 à 6.

10. Matière servant à l'enregistrement sensible à l'effet de la pression, selon la revendication 9, caractérisée en ce qu'elle renferme le phtalide dissous dans un solvant organique ainsi qu'au moins une substance solide qui accepte les électrons.